**Europäisches Patentamt**

**European Patent Office**

**Office européen des brevets**

(11) Numéro de publication : **0 334 744 B1**

(12) **FASCICULE DE BREVET EUROPEEN**

(19)

(45) Date de publication du fascicule du brevet :
**16.09.92 Bulletin 92/38**

(21) Numéro de dépôt : **89400784.8**

(22) Date de dépôt : **21.03.89**

(51) Int. Cl.⁵ : **G01N 33/49, B01L 3/02**

(54) **Dispositif de déclenchement d'un circuit de mesure d'un paramètre de réaction au moment de la distribution d'une dose de réactif à l'aide d'une pipette-réservoir.**

(30) Priorité : **22.03.88 FR 8804334**

(43) Date de publication de la demande :
**27.09.89 Bulletin 89/39**

(45) Mention de la délivrance du brevet :
**16.09.92 Bulletin 92/38**

(84) Etats contractants désignés :
**DE ES IT**

(56) Documents cités :
**EP-A- 0 125 624
DE-A- 1 598 123
FR-A- 2 571 497
MESSEN + PRÜFEN/AUTOMATIK, juillet/août
1979, Hans Holzmann-Verlag, Bad Wörishofen,
DE; G.PELTZ, pp. 551-555**

(73) Titulaire : **DIAGNOSTICA STAGO (société
anonyme)
9 rue des Frères Chausson
F-92602 Asnières (FR)**

(72) Inventeur : **Leviet, Hong
9, rue du Berceau
F-95870 Bezons (FR)**
Inventeur : **Vilain, Pascal
27, rue Croix du Bellay
F-67240 Hadancourt le Haut Clocher (FR)**
Inventeur : **Martinoli, Jean-Luc
2, rue du Tremble
F-92390 Villeneuve la Garenne (FR)**

(74) Mandataire : **de Saint-Palais, Arnaud Marie
CABINET MOUTARD 35, Avenue Victor Hugo
F-78180 Voisins le Bretonneux (FR)**

EP 0 334 744 B1

## Description

L'invention s'applique aux appareils de mesure d'un paramètre de réaction et, en particulier, aux appareils de mesure d'un temps de réaction, par exemple le temps de coagulation d'un plasma sanguin ou, plus généralement, aux appareils de mesure d'une autre grandeur physique (par exemple, une densité optique), qui varie en fonction du temps au cours d'une réaction.

Dans ces appareils, l'opérateur distribue des doses prédéterminées d'un liquide contenant le ou les réactifs nécessaires dans une pluralité de godets disposés, à l'intérieur d'une zone délimitée, dans un plateau que comporte l'appareil de mesure.

Cette distribution s'effectue généralement au moyen d'une pipette-réservoir comportant un poussoir dont la manoeuvre entraîne un piston et provoque ainsi la distribution d'une dose. Après chaque manoeuvre, le poussoir est rappelé dans sa position de repos et la pipette permet ainsi de distribuer rapidement plusieurs doses successives dans les godets respectifs. Il faut évidemment que les organes de mesure du paramètre de réaction soient informés des instants de distribution respectifs, c'est-à-dire qu'un signal de synchronisation soit fourni à chaque organe de mesure au début de la réaction.

La solution la plus courante consiste en ce que l'opérateur déclenche des chronomètres avec une main au moment où il actionne le poussoir avec l'autre main.

Pour améliorer la précision et simplifier la tâche de l'opérateur, on a proposé d'utiliser des circuits électroniques de mesure du temps et de les déclencher par des tops fournis par un système capteur.

Une première solution connue consiste à utiliser comme capteur une barrière optique détectant la sortie du jet de liquide à l'extrémité de la pipette. Une deuxième solution consiste à disposer à l'intérieur de la pipette un interrupteur actionné par le mouvement du poussoir et inséré dans un circuit électrique générateur de signaux relié par fil aux circuits de mesure.

Ces solutions sont relativement compliquées à mettre en oeuvre et/ou ne garantissent pas que le liquide ait effectivement été introduit dans le godet au moment du déclenchement.

Un déclenchement intempestif peut, en particulier, se produire lorsque l'opérateur rejette la dernière fraction de dose non utilisable.

L'invention a pour objet un système utilisant une pipette munie d'un capteur du déplacement du poussoir, mais exempt des inconvénients ci-dessus.

Suivant l'invention, ledit capteur est inséré dans le circuit d'un dispositif d'émission d'ondes sous la forme d'un signal d'identification, ledit dispositif étant associé à la pipette, et un dispositif récepteur-identificateur, associé à l'appareil de mesure du temps, reçoit ledit signal, lorsqu'il est placé dans le champ d'émission des ondes, tandis qu'un détecteur volumétrique de la présence de la pipette dans la zone de support des godets fournit un signal de validation des tops de déclenchement engendrés par le dispositif récepteur-identificateur.

Cette solution assure la fiabilité de la transmission des ondes entre la pipette et l'appareil de mesure en permettant l'utilisation d'un émetteur couvrant un champ suffisamment large pour ne pas faire dépendre la réception de l'orientation précise que l'opérateur donne à la pipette, sans pour cela faire courir le risque d'un déclenchement intempestif des circuits de mesure à un moment où la pipette n'est pas effectivement placée en position de distribution du réactif dans un godet.

D'autres particularités, ainsi que les avantages de l'invention, apparaîtront clairement à la lumière de la description ci-après.

Au dessin annexé :

La figure l représente une pipette équipée d'un capteur et d'un dispositif d'émission d'ondes, ainsi que les circuits de réception et de validation des tops de déclenchement associés à l'appareil de mesure ;

La figure 2 est une vue en perspective du boîtier de l'appareil de mesure, équipé du détecteur volumétrique ; et

La figure 3 est une vue de dessus dudit boîtier.

A la figure l, on a représenté une pipette-réservoir l ayant un doigt de manoeuvre l0l qui commande, de façon connue en soi, le déplacement d'un piston, non figuré, pour expulser une dose du liquide contenu dans le réservoir, et revient immédiatement dans sa position initiale.

Au doigt l0l est relié un capteur l02, que l'on a représenté symboliquement sous la forme d'un interrupteur mécanique, agencé pour fermer un circuit électronique à chaque manoeuvre du doigt l0l.

Ce circuit comprend une source d'énergie, par exemple une pile l03, un codeur d'identification l04 et un ensemble d'organes émetteurs l05 agencés pour que le champ d'émission présente une symétrie de révolution par rapport à l'axe longitudinal de la pipette (ce champ d'émission a été symbolisé par deux lignes en trait mixte).

Dans le mode d'exécution préféré décrit, les organes émetteurs sont constitués par des diodes électroluminescentes qui émettent un signal optique (de préférence infrarouge).

Un dispositif récepteur comportant une photodiode 2 et un décodeur d'identification 3 est associé à l'appareil de mesure. Lorsque l'opérateur place la pipette en position de distribution, qu'il soit droitier ou gaucher, une partie du champ émis sera dirigée vers le dispositif récepteur.

Le codeur l04 effectue un codage du signal optique propre à l'identification de la pipette : à titre d'exemple, le signal optique pourra être codé en impulsions ayant une fréquence de récurrence déterminée, mais il doit être bien compris que l'invention ne porte pas sur le moyen d'identification utilisé, pas plus que sur le type d'onde d'émission, l'utilisation d'une émission radioélectrique ou même d'ultrasons pouvant être envisagée. De même, la réalisation du capteur l02 pourrait mettre en oeuvre différents moyens connus, tels que : dispositif à effet Hall, barrière optique, dispositif inductif, capacitif ou magnétorésistif, relais ILS, ou autres.

A la réception du signal par l'organe 2, le décodeur 3 transmet, à sa sortie 3l, reliée par l'intermédiaire d'une porte ET 32 au dispositif de mesure du temps de réaction (qui mesure par exemple, le temps qui s'écoule entre l'instant d'introduction d'un réactif dans un godet 4l contenant du plasma et l'instant de détection, par l'appareil de mesure, de la coagulation du plasma), un top de déclenchement de la mesure du temps.

Le contenu de la pipette-réservoir ne correspondant généralement pas à un nombre entier de doses, à un certain moment de la distribution des doses dans les godets du dispositif de mesure, il reste dans la pipette une fraction de dose de réactif qu'il est nécessaire de rejeter avant d'aspirer une nouvelle quantité.

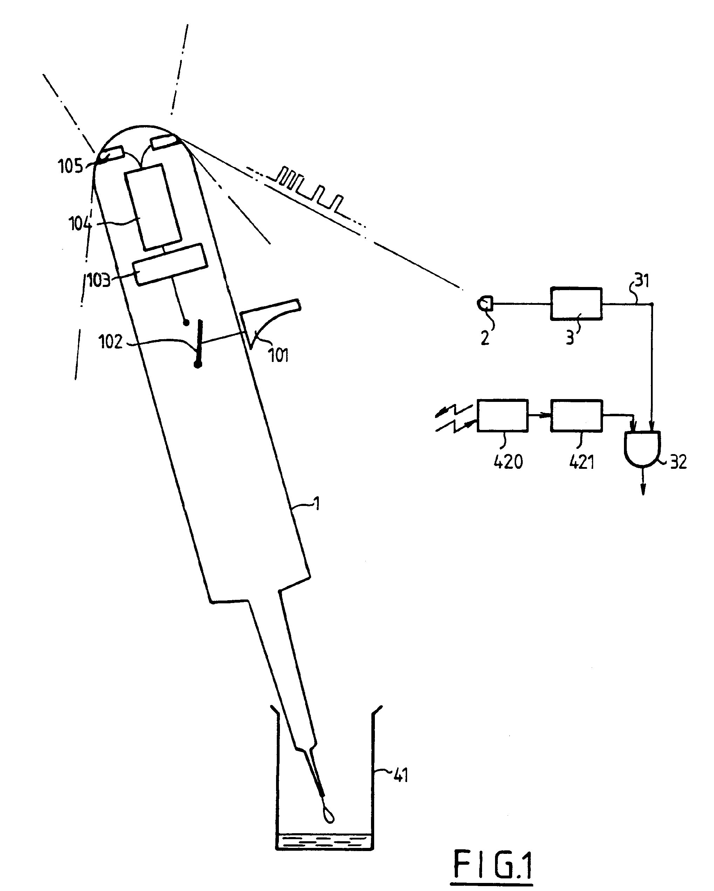

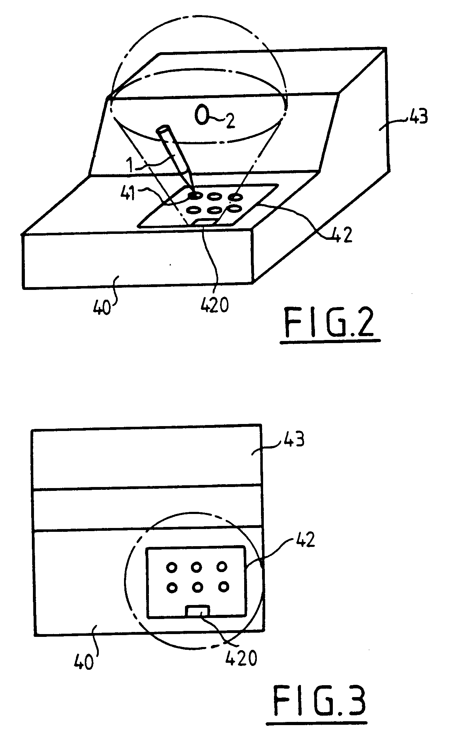

Pour éviter que la manoeuvre de rejet, ou tout autre manipulation qui placerait le récepteur 2 dans le champ d'émission sans que la pipette se trouve en position appropriée de distribution, ne déclenche le dispositif de mesure du temps, il est prévu, selon l'invention, un dispositif de validation représenté aux figures 2 et 3.

Celles-ci représentent le boîtier d'un dispositif de mesure comportant un plateau 40 qui porte les godets, tels que 4l, situés dans une zone de distribution figurée comme étant un rectangle 42 et un pupitre 43. Un détecteur volumétrique à infrarouge 420 est placé dans la zone de distribution, en dehors de l'espace occupé par les godets, mais agencé et disposé pour que son champ d'émission-réception, symbolisé par une ligne en trait mixte, couvre l'ensemble des zones susceptibles d'être balayées par le bras d'un opérateur, gaucher ou droitier, lorsque celui-ci distribue les doses dans les godets.

Pendant la distribution, le détecteur 420 détecte donc l'interception de son volume de surveillance par le bras de l'opérateur et transmet à la porte ET 32 un signal de validation du top de déclenchement lorsqu'un seuil, défini par un circuit à seuil 42l, a été dépassé.

Au moment du rejet de la fraction de dose inutilisable, le bras de l'opérateur n'intercepte pas le champ du détecteur 42 et, par conséquent, la validation ne se produit pas. Le réglage du seuil permet de limiter le volume de détection pour éviter des déclenchements intempestifs.

La position du récepteur 2 sur la façade du pupitre 43 étant située dans le volume d'émission du détecteur 420, il faut évidemment que l'émission du détecteur de surveillance 420 ne soit pas de nature à influencer le récepteur 2.

En variante, le détecteur 420 pourrait comporter uniquement qu'un récepteur sensible à l'émission des émetteurs l05 lorsqu'elle est réfléchie par la pipette. Les champs de réception des dispositifs 2 et 420 devraient alors se croiser au moment où la pipette serait placée en position de distribution au-dessus de l'un des godets.

Quels que soient la nature du rayonnement utilisé et les agencements relatifs du détecteur de surveillance et du récepteur de déclenchement, il est clair que le dispositif évitera un déclenchement indésiré des circuits de mesure du temps par une manoeuvre de l'opérateur ne correspondant pas à la distribution effective d'une dose.

## Revendications

l. Dispositif de déclenchement d'un circuit de mesure d'un paramètre de réaction évolutif en fonction du temps au moment de la distribution d'une dose de réactif à l'aide d'une pipette-réservoir, comportant un capteur associé à la pipette et sensible au déplacement du poussoir de manoeuvre de celle-ci, caractérisé en ce que ledit capteur (l02) est inséré dans le circuit d'un dispositif (l05) d'émission d'ondes sous la forme d'un signal d'identification, ledit dispositif étant associé à la pipette, et qu'un dispositif récepteur-identificateur (2-3), associé à l'appareil de mesure, reçoit ledit signal, lorsqu'il est placé dans le champ d'émission des ondes, tandis qu'un détecteur volumétrique (420) de la présence de la pipette dans la zone de support des godets fournit un signal de validation des tops de déclenchement engendrés par le dispositif récepteur-identificateur.

2. Dispositif selon la revendication l, caractérisé en ce que le champ du dispositif d'émission présente une symétrie de révolution par rapport à l'axe longitudinal de la pipette.

3. Dispositif selon la revendication l ou 2, caractérisé en ce que ledit détecteur volumétrique comporte un organe à seuil réglable (42l).

4. Dispositif selon la revendication 2 ou 3, caractérisé en ce que les tops de déclenchement sont transmis à l'appareil de mesure par une porte ET (32) validée par les signaux de sortie du détecteur volumétrique.

## Patentansprüche

1. Auslösevorrichtung für eine Schaltung zur Messung eines zeitlich veränderlichen Kennwertes im Augenblick der Eingabe einer Reagenzstoffdosis, mittels einer Vorratspipette, mit einem, der Pipette zugeordneten, Sensor, der auf eine Verschiebung ihres Betätigungskolbens anspricht, dadurch gekennzeichnet, dass besagter Sensor (102) in den Schaltkreis einer Vorrichtung (105) zur Abgabe von Wellen in Form eines Identifiziersignales eingeordnet ist und diese Vorrichtung der Pipette zugeordnet ist, und dass eine dem Messgerät zugeordnete Empfangs- und Identifiziervorrichtung (2-3) besagtes Signal erhält, wenn sie sich im Wellenemissionsbereich befindet, während ein Volumendetektor (420), welcher das Vorhandensein der Pipette in der Trägerzone für die Behälter erfasst, ein Gültigmachungssignal für die von der Empfangs- und Identifiziervorrichtung erzeugten Auslöseimpulse liefert.

2. Vorrichtung nach Anspruch 1,
dadurch gekennzeichnet, dass das Feld der Emissionsvorrichtung im Verhältnis zur Längsachse der Pipette drehsymmetrisch ist.

3. Vorrichtung nach Anspruch 1 oder 2,
dadurch gekennzeichnet, dass besagter Volumendetektor ein Organ (420) mit einstellbarem Schwellenwert aufweist.

4. Vorrichtung nach Anspruch 2 oder 3,
dadurch gekennzeichnet, dass die Auslöseimpulse dem Messgerät mittels eines durch die Ausgangssignale des Volumendetektors gültiggemachten UND-Gates (32) übermittelt werden.

**Claims**

1. Device for triggering a circuit for measuring a reaction parameter which varies in time, at the time of distributing a dose of reagent by means of a pipette, comprising a sensor which is associated with the pipette and responsive to a movement of the operating pusher of said pipette, characterized in that said sensor (102) is inserted in the circuit of a device (105) which emits waves in the form of an identification signal, said device being associated with the pipette, and in that a wave receiving and identification device (2-3) which is associated with the measuring device, receives said signal when it is placed in the field of emission of the waves, whereas a volumetric detector (420) which detects the presence of the pipette in the support zone of the cups supplies a signal for validating the triggering beeps generated by the wave receiving and identification device.

2. Device according to claim 1,
characterized in that the field of the emission device is symmetrically distributed around the longitudinal axis of the pipette.

3. Device according to claim 1 or 2,
characterized in that said volumetric detector comprises an adjustable threshold member (421).

4. Device according to claim 2 or 3,
characterized in that the triggering beeps are transmitted to the measuring device by an AND gate (32) validated by the output signals of the volumetric detector.

F I G.1

FIG.2

FIG.3